# EUROPÄISCHE PATENTSCHRIFT

(11) **EP 2 834 347 B1**
(45) Veröffentlichungstag und Bekanntmachung des Hinweises auf die Patenterteilung: **28.02.2018**
(21) Anmeldenummer: 13715159.3
(22) Anmeldetag: 25.03.2013
(51) Int. Cl.: C12N 1/21, C12P 13/06, C12P 13/00

(54) **VERFAHREN ZUR AEROBEN HERSTELLUNG VON ALANIN**
METHOD FOR AEROBIC PRODUCTION OF ALANINE
PROCÉDÉ DE FABRICATION AÉROBIE D'ALANINE

(30) Priorität: 02.04.2012 EP 12162846
(43) Veröffentlichungstag der Anmeldung: 11.02.2015
(73) Patentinhaber: Evonik Degussa GmbH, 45128 Essen (DE); Technische Universität München, 80290 München (DE)
(72) Erfinder: HENNEMANN, Hans-Georg, 45770 Marl (DE); SCHAFFER, Steffen, 45699 Herten (DE); WESSEL, Mirja, 44799 Bochum (DE); PÖTTER, Markus, 201702 Shangai (CN); PFEFFER, Jan Christoph, 63452 Hanau (DE); HAAS, Thomas, 48161 Münster (DE); CORTHALS, Jasmin, 44879 Bochum (DE); ECKL, Eva-Maria, 45768 Marl (DE); ROEDER, Silvana, 79249 Merzhausen (DE); KROUTIL, Wolfgang, 8042 Graz (AT); SKERRA, Arne, 85354 Freising (DE)
(86) Internationale Anmeldenummer: PCT/EP2013/056190
(87) Internationale Veröffentlichungsnummer: WO 2013/149864

(56) Entgegenhaltungen:
- WO-A1-2009/077461
- WO-A1-2011/131420
- WO-A2-2013/024114
- XUELI ZHANG ET AL: "Production of l-alanine by metabolically engineered Escherichia coli", APPLIED MICROBIOLOGY AND BIOTECHNOLOGY, Bd. 77, Nr. 2, 15. September 2007 (2007-09-15), Seiten 355-366, XP019560700, ISSN: 1432-0614
- GEOFFREY M SMITH ET AL: "Fed-batch two-phase production of alanine by a metabolically engineered Escherichia coli", BIOTECHNOLOGY LETTERS, Bd. 28, Nr. 20, 11. August 2006 (2006-08-11), Seiten 1695-1700, XP019391629, ISSN: 1573-6776
- M. LEE ET AL: "Aerobic production of alanine by Escherichia coli aceF ldhA mutants expressing the Bacillus sphaericus alaD gene", APPLIED MICROBIOLOGY AND BIOTECHNOLOGY, Bd. 65, Nr. 1, 1. Juli 2004 (2004-07-01), XP055034646, ISSN: 0175-7598

## Beschreibung

Die Erfindung betrifft ein Verfahren zur Herstellung von Alanin umfassend (a) Bereitstellen einer eine rekombinante Alanin-Dehydrogenase exprimierenden Zelle, (b) Kultivieren der Zelle unter aeroben Bedingungen in Anwesenheit einer anorganischen Stickstoffquelle in einer wässrigen Phase, und (c) Kontaktieren der Zelle mit einer hydrophoben organischen Phase, wobei es sich bei der Zelle um eine prokaryontische oder eine niedere eukaryontische Zelle handelt.
Alanin findet neben der Funktion als proteinogene Aminosäure, d.h. als Aminosäure, die einen essentiellen Bestandteil von zahlreichen Proteinen darstellt, als weitverbreiteter Amindonor für biotechnologische Verfahren und chemische Synthesen Verwendung. Im Fall industrieller biotechnologischer Synthesen kann das benötigte Alanin den biosynthetisch aktiven Zellen in Form von chemisch synthetisiertem Alanin oder in Form von Hefeextrakten zugegeben werden. Mit Hinblick auf Kostenaspekte und eventuell die Reinheit eines aus dem Kulturmedium aufzureinigenden Produktes ist es jedoch günstiger, wenn die biosynthetisch aktiven Zellen das benötigte Alanin selbst in ausreichender Menge herstellen. Insofern besteht ein hohes Interesse an biotechnologisch einsetzbaren Mikroorganismen, die kontinuierlich eine möglichst hohe Menge an Alanin produzieren, das dann für nachfolgende Syntheseschritte innerhalb oder außerhalb der Zelle zur Verfügung steht.
Der Stand der Technik lehrt, dass eine besonders große Menge an Alanin gebildet wird, wenn eine Alanin-Dehydrogenase exprimierende Zelle unter anaeroben Bedingungen gehalten wird ("Production of L-alanine by metabolically engineered Escherichia coli" Appl Microbiol Biotechnol (2007), 77:355-366). Derartige Bedingungen bedingen jedoch ein deutlich reduziertes Wachstum der Zellen, eine verringerte Stoffwechselleistung und die Verstoffwechselung teurer Substrate wie Glucose zu unverwünschten Nebenprodukten des Primärstoffwechsels auf Kosten der Effizienz und der Produktausbeute des Gesamtprozesses. Geoffrey M Smith (2006), Biotechnology letters, 28(20): 1695-1700 und M Lee et al. (2004), Applied Microbiology and Biotechnology, 65(1): 56-60) lehren, dass Alanin gebildet wird, wenn eine Alanin-Dehydrogenase exprimierende Zelle unter aeroben Bedingungen gehalten wird.

Aber, die Produktion ist gering. Für die Entwicklung wirtschaftlicher biotechnologischer Prozesse im Industriemaßstab ist die Möglichkeit, diese unter aeroben Bedingungen durchzuführen, daher essentiell.

Vor diesem Hintergrund besteht Bedarf an Verfahren, die geeignet sind, die in einer biotechnologisch relevanten Zelle für Synthesen zur Verfügung stehende Alaninmenge *unter aeroben Bedingungen* erhöhen.

Weiterhin besteht Bedarf an Verfahren zur biotechnologischen Herstellung von ω-Aminocarbonsäuren unter verringerter oder möglichst lediglich optionaler oder nur zeitweilig notwendiger Zugabe von Alanin, wobei der zur Herstellung der ω-Aminocarbonsäure benötigte Stickstoff in Form von anorganischen Stickstoffsalzen bereitgestellt wird, und/oder Verfahren, bei denen die Ausbeute an ω-Aminocarbonsäure bei einem Reaktionsmodus ohne Zugabe von Alanin verbessert ist.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Das der Erfindung zu Grunde liegende Problem wird in einem ersten Aspekt gelöst durch ein Verfahren zur Herstellung von Alanin umfassend
a) Bereitstellen einer eine rekombinante Alanin-Dehydrogenase exprimierenden Zelle,
b) Kultivieren der Zelle unter aeroben Bedingungen in Anwesenheit einer anorganischen Stickstoffquelle in einer wässrigen Phase, und
c) Kontaktieren der Zelle mit einer hydrophoben organischen Phase,
wobei es sich bei der Zelle um eine prokaryontische oder eine niedere eukaryontische Zelle handelt.

In einer ersten Ausführungsform des ersten Aspekts wird das Problem durch ein Verfahren gelöst, wobei die Zelle eine Nukleinsäure umfassend eine für ein alkL-Polypeptid, bevorzugt das AlkL von *Pseudomonas putida* (Datenbankcode AJ245436) oder eine Variante davon, kodierende Sequenz und/oder das alkL-Polypeptid oder eine Variante davon aufweist.

In einer zweiten Ausführungsform, die auch eine Ausführungsform der ersten Ausführungsform darstellt, wird das Problem durch ein Verfahren gelöst, wobei das alkL-Polypeptid oder die Variante davon heterolog ist und bevorzugt überexprimiert wird.

In einer dritten Ausführungsform, die auch eine Ausführungsform der ersten bis zweiten Ausführungsform darstellt, wird das Problem durch ein Verfahren gelöst, wobei Schritt c) dadurch verwirklicht wird, dass Schritt b) in Anwesenheit der organischen Phase durchgeführt wird.

In einer vierten Ausführungsform, die auch eine Ausführungsform der ersten bis dritten Ausführungsform darstellt, wird das Problem durch ein Verfahren gelöst, wobei die organische Phase einen hydrophoben Fettsäureester umfasst, bevorzugt Laurinsäuremethylester.

In einer fünften Ausführungsform, die auch eine Ausführungsform der vierten Ausführungsform darstellt, wird das Problem durch ein Verfahren gelöst, wobei die organische Phase neben dem hydrophoben Fettsäureester eine hydrophobe Fettsäure umfasst.

In einer sechsten Ausführungsform, die auch eine Ausführungsform der ersten bis fünften Ausführungsform darstellt, wird das Problem gelöst durch ein Verfahren, wobei die organische Phase wenigstens ein hydrophobes Lösungsmittel aus der Gruppe umfassend unsubstituierte, substituierte, verzweigte und unverzweigte Alkane, Cycloalkane, Aryle, Heteroaryle, Dialkylether, Fettalkohole, Triglyceride und Halogenkohlenwasserstoffe umfasst.

In einer siebten Ausführungsform, die auch eine Ausführungsform der sechsten Ausführungsform darstellt, wird das Problem gelöst durch ein Verfahren, wobei die hydrophobe Fettsäure eine ungesättigte Fettsäure ist, bevorzugt Ölsäure.

In einer achten Ausführungsform, die auch eine Ausführungsform der ersten bis siebten Ausführungsform darstellt, wird das Problem gelöst durch ein Verfahren, wobei die Zelle eine heterologe Transaminase aufweist, die Alanin als Substrat erkennt, und die Transaminase bevorzugt aus der Gruppe ausgewählt ist, die die ω-Transaminase aus *Chromobacterium violaceum* ATCC 12472 (Datenbankcode NP_901695) sowie Varianten davon umfasst.

In einer neunten Ausführungsform, die auch eine Ausführungsform der ersten bis achten Ausführungsform darstellt, wird das Problem gelöst durch ein Verfahren, wobei die Zelle zusätzlich zur heterologen Transaminase eine oder mehr als eine Monooxygenase aufweist, die allein oder sequentiell die Oxidation einer Fettsäure oder eines Fettsäureesters zur ω-Oxo-Fettsäure oder zum ω-Oxo-Fettsäureester katalysiert.

In einer zehnten Ausführungsform, die auch eine Ausführungsform der ersten bis neunten Ausführungsform darstellt, wird das Problem gelöst durch ein Verfahren, wobei die eine oder mehr als eine Monooxygenase heterolog ist und bevorzugt aus der Gruppe ausgewählt ist, die Monooxygenasen aus der AlkB-Familie und Cytochrom P450-Monoxygenasen der CYP153-Familie und Varianten davon umfasst.

In einer elften Ausführungsform, die auch eine Ausführungsform der ersten bis zehnten Ausführungsform darstellt, wird das Problem gelöst durch ein Verfahren, wobei es sich bei der Zelle um eine bakterielle Zelle handelt, bevorzugt um *Escherichia coli.*

In einer zwölften Ausführungsform, die auch eine Ausführungsform der ersten bis elften Ausführungsform darstellt, wird das Problem gelöst durch ein Verfahren, wobei Schritt c) wenigstens 60 Minuten andauert.

In einer dreizehnten Ausführungsform, die auch eine Ausführungsform der ersten bis zwölften Ausführungsform darstellt, wird das Problem gelöst durch ein Verfahren, wobei die wässrige Phase weniger als 10 mM, bevorzugt weniger als 5 mM, noch bevorzugter noch weniger als 1 mM Alanin umfasst.

In einer vierzehnten Ausführungsform, die auch eine Ausführungsform der ersten bis dreizehnten Ausführungsform darstellt, wird das Problem gelöst durch ein Verfahren, wobei die organische Phase wenigstens 5 Volumenprozent, bevorzugt mehr als 20 Volumenprozent der Summe der Volumina von wässriger und organischer Phase ausmacht.
In einem zweiten Aspekt wird das der vorliegenden Erfindung zu Grunde liegende Problem gelöst durch eine Verwendung einer eine heterologe Alanin-Dehydrogenase exprimierenden Zelle, zur Herstellung von Alanin unter aeroben Bedingungen in Anwesenheit einer hydrophoben organischen Phase, wobei es sich bei der Zelle um eine prokaryontische oder eine niedere eukaryontische Zelle handelt und wobei die organische Phase bevorzugt einen hydrophoben Fettsäureester und eine hydrophobe Fettsäure, noch bevorzugter Laurinsäuremethylester und Ölsäure umfasst.
In einem dritten Aspekt wird das der vorliegenden Erfindung zu Grunde liegende Problem gelöst durch die Verwendung einer hydrophoben organischen Phase zur Erhöhung der Ausbeute an Alanin umfassend Kontaktieren einer Alanin herstellenden prokaryontischen oder einer niederen eukaryontischen Zelle mit der hydrophoben organischen Phase, wobei die organische Phase bevorzugt einen hydrophoben Fettsäureester und eine hydrophobe Fettsäure, noch bevorzugter Laurinsäuremethylester und Ölsäure umfasst.
In einer weiteren Ausführungsform des ersten, zweiten oder dritten Aspektes wird das Problem gelöst durch ein Verfahren oder eine Verwendung, wobei die Zelle in einer wässrigen Lösung bei einer optischen Dichte von wenigstens 0,5, bevorzugter wenigstens 1, noch bevorzugter wenigstens 5, am bevorzugtesten wenigstens 10 vorliegt.
In einer weiteren Ausführungsform des ersten, zweiten oder dritten Aspekts wird das Problem gelöst durch ein Verfahren oder eine Verwendung, wobei es sich bei der Alanin-Dehydrogenase um die Alanin-Dehydrogenase aus *Bacillus subtilus* (Datenbankcode P_391071) oder um eine Variante davon handelt.
Die Erfinder haben herausgefunden, dass die Alaninproduktion durch eine prokaryontische, Alanin-Dehydrogenase exprimierende Wirtszelle unter aeroben Bedingungen und in Anwesenheit einer anorganischen Stickstoffquelle überraschend deutlich erhöht ist, wenn die Zelle mit einer hydrophoben organischen Lösung kontaktiert wird.

Die erfindungsgemäße Lehre kann zur Verbesserung sämtlicher biotechnologischer Verfahren herangezogen werden, die die Herstellung von Produkten wie Feinchemikalien unter Verbrauch von Alanin und bei Verwendung einer stoffwechselaktiven Zelle, deren Kultivierung in einem wässrigen Medium und die Aufarbeitung des Produktes umfassen. In einer bevorzugten Ausführungsform wird unter dem Begriff "Zelle", wie hierin verwendet, eine lebende Zelle mit Stoffwechselaktivität verstanden, bevorzugt ein Ganzzellkatalysator, die ein für die biotechnologische Herstellung des interessierenden Produktes relevantes Enzym in aktiver Form exprimiert oder noch bevorzugter überexprimiert. Bei der Zelle kann es sich um einen Prokaryonten, einschließlich Archaeen, oder um einen Eukaryonten handeln, im Falle eines Prokaryonten bevorzugt aus der Gruppe von Gattungen umfassend *Pseudomonas*, *Corynebacterium* und *Escherichia.* In einer noch bevorzugteren Ausführungsform handelt es sich bei der Zelle um eine bakterielle Zelle, noch bevorzugter um eine Gram-negative bakterielle Zelle, am bevorzugtesten um *E. coli.* In einer weiteren bevorzugten Ausführungsform handelt es sich um eine eukaryontische Zelle, bevorzugter um eine niedere eukaryontische Zelle bzw. eine Pilzzelle, noch bevorzugter um eine Hefezelle, am bevorzugtesten um *Saccharomyces* oder *Candida, Pichia, insbesondere Candida tropicalis.* In einer bevorzugten Ausführungsform bedeutet der Begriff "niederer Eukaryont", wie hierin verwendet, ein in sämtlichen Phasen seiner Existenz unizellulärer Eukaryont, im Gegensatz zu höheren Eukaryonten, die den überwiegenden Teil ihres Lebens in Form eines mehrzelligen Organismus mit Geweben umfassend differenzierte Zellen verbringen. Der Begriff "Zelle" wird, in einer besonderen Ausführungsform, in dieser Anmeldung gleichbedeutend und austauschbar mit dem Begriff "Mikroorganismus" verwendet. Weiterhin kann es sich bei der Zelle um eine isolierte Zelle oder eine Mischung verschiedener Zellen handeln.

Voraussetzung für die Benutzung der erfindungsgemäßen Lehre ist das Vorliegen einer wässrigen Phase, d. h. einem wässrigen Kultur- oder Reaktionsmedium, das zur wenigstens zeitweiligen Erhaltung oder Kultivierung der Zelle geeignet ist. Dem Fachmann sind zahlreiche wässrige Kulturmedien bekannt, die für die Erhaltung oder Kultivierung von Zellen, insbesondere biotechnologisch bedeutsamer Zellen, geeignet sind. Darunter fallen gleichermaßen Vollmedien wie LB-Medien, Minimalmedien wie M9-Medien sowie Selektivmedien, beispielsweise solche, die eine hohe Salzkonzentration enthalten und daher nur das Wachstum halophiler oder zumindest halotoleranter Organismen ermöglichen. In einer bevorzugten Ausführungsform wird unter dem Begriff "wässrige Phase", wie hierin verwendet, ein mit hydrophoben Lösungsmitteln im Wesentlichen unmischbares Reaktions- oder Kulturmedium auf Wasserbasis verstanden, das mit Hinblick auf sämtliche relevante Faktoren, insbesondere pH-Wert, Salzgehalt und Temperatur, derart beschaffen ist, das es die Lebensfähigkeit darin enthaltener Zellen, bevorzugt Mikroorganismen, wenigstens zeitweilig erhält oder fördert und sowohl wässriges Kulturmedium als auch hydrophobe organische Phase bei üblichen Inkubationstemperaturen für biotechnologisch nützliche Organismen, bevorzugt bei 25 °C, in flüssiger Form vorliegen. Die Temperaturansprüche verschiedener biotechnologisch bedeutsamer Zellen können mikrobiologischen und molekularbiologischen Lehrbüchern entnommen werden, z.B. Fuchs/Schlegl, 2008. In einer bevorzugten Ausführungsform liegt der pH-Wert des wässrigen Kulturmediums zum Zeitpunkt des Kontaktierens zwischen 4 bis 9, bevorzugter zwischen 4,5 bis 8,5, am bevorzugtesten zwischen 6,5 und 7,5. In einer weiteren bevorzugten Ausführungsform liegt die Temperatur zwischen 5 und 42 °C, bevorzugter zwischen 15 und 40 °C, am bevorzugtesten zwischen 20 und 37 °C.

Die erfindungsgemäße Lehre sieht vor, dass die Zelle eine rekombinante Alanin-Dehydrogenase exprimiert. In einer bevorzugten Ausführungsform wird unter dem Begriff "Alanin-Dehydrogenase", wie hierin verwendet, ein Enzym verstanden, das die Umwandlung von Pyruvat, Ammoniak und NADH oder deren Salze zu L-Alanin, Wasser und NAD⁺ katalysiert. Bevorzugt handelt es sich bei der Alanindehydrogenase um eine intrazelluläre Alanindehydrogenase, noch bevorzugter um eine rekombinante intrazelluläre Alanindehydrogenase eines bakteriellen Ganzzellkatalysators. In einer besonders bevorzugten Ausführungsform handelt es sich um das Enzym von *Bacillus subtilis* (Datenbankcode P_391071) oder Varianten davon. Weitere Beispiele umfassen die Enzyme aus *Rhizobium leguminosarum* (Datenbankcode YP_002975437), *Bacillus megaterium* (Datenbankcode YP_003565624), *Rhodobacter capsulatus* (Datenbankcode ADE84249.1) und *Bacillus subtilis* (Datenbankcode NP_391071) und Varianten davon.

Für die Alaninsynthese ist essentiell, dass die Edukte in ausreichender Menge vorliegen, insbesondere eine anorganische Stickstoffquelle. In einer bevorzugten Ausführungsform ist unter dem Begriff "anorganische Stickstoffquelle", wie hierin verwendet, ein anorganisches stickstoffhaltiges Salz zu verstehen, das Ammonium umfasst oder im Stoffwechsel der Zelle zu Ammonium umgewandelt werden kann. Beispiele umfassen Ammoniumchlorid, Ammoniumnitrat, Ammoniumsulfat, Ammoniumhydroxid, Ammoniumphosphat, Ammoniumcarbonat und dergleichen. In einer bevorzugten Ausführungsform beträgt die Konzentration von Ammonium im Medium 0,05 bis 5, bevorzugter 0,1 bis 3, am bevorzugtesten 0,5 bis 3 g/L. Bevorzugt wird die anorganische Stickstoffquelle der Zelle erfindungsgemäß dadurch bereitgestellt, dass die entsprechende Verbindung in ausreichender Menge zur wässrigen Phase gegeben wird.

In einer bevorzugten Ausführungsform weist die Zelle neben der rekombinanten Alanin-Dehydrogenase wenigstens ein weiteres rekombinantes Enzym auf. In einer bevorzugten Ausführungsform handelt es sich dabei um ein Enzym, das NAD(P)⁺ zu NAD(P)H reduziert, beispielsweise eine Monooxygenase der Cytochrom P450-Monooxygenase der CYP153-Familie oder der AlkBGT-Familie oder eine Alkoholdehydrogenase. Besonders vorteilhaft ist daher die Verwendung eines Systems, bei dem das NAD(P)⁺ reduzierende Enzym und die Aminosäuredehydrogenase den gleichen Redoxcofaktor, bevorzugt NAD(H) oder NADP(H), umsetzen. NADP-abhängige Alanindehydrogenasen umfassen das Enzym aus *Rhodobacter capsulatus* (Datenbankcode ADE84249.1) und Varianten davon. NAD-abhängige Alanindehydrogenasen umfassen die Alanindehydrogenase aus *Bacillus subtilis subsp. subtilis* str. 168 (Datenbankcode NP_391071) und Varianten davon.

In einer besonders bevorzugten Ausführungsform handelt es sich bei der Monooxygenase um eine Monooxygenase aus der AlkB-Familie. AlkB stellt eine Oxidoreduktase aus dem AlkBGT-System aus *Pseudomonas putida* dar, die für ihre Hydroxylase-Aktivität bekannt ist. Diese ist von zwei weiteren Polypeptiden, AlkG und AlkT abhängig. AlkT wird als FAD-abhängige Rubredoxin-Reduktase charakterisiert, die Elektronen von NADH auf AlkG überträgt. AlkG ist ein Rubredoxin, ein eisenhaltiges Redoxprotein, das als direkter Elektronendonor für AlkB fungiert. In einer bevorzugten Ausführungsform wird unter dem Begriff "Monooxygenase aus der alkB-Familie", wie hierein verwendet, eine membranständige Alkanhydroxylase verstanden. In einer weiteren bevorzugten Ausführungsform wird unter dem selben Begriff "Alkanhydroxylase des alkB-Typs" ein Polypeptid mit einer Sequenzhomologie von zunehmend bevorzugt wenigstens 75, 80, 85, 90, 92, 94, 96, 98 oder 99 % zur Sequenz des AlkB von *Pseudomonas putida* Gpo1 (Datenbankcode: CAB54050.1, dieser Datenbankcode und sämtliche weitere in diesem Dokument verwendete Datenbankcodes stammen aus der Genbank Protein-Datenbank des NCBI in dem am 9. November 2011 verfügbaren Release) verstanden. Der Begriff "Sequenz", wie hierin verwendet, kann sich auf die Aminosäuresequenz eines Polypeptides und/oder die dafür codierende Nukleinsäuresequenz beziehen.

In einer besonders bevorzugten Ausführungsform handelt es sich bei der Monooxygenase um Cytochrom P450-Monooxygenase der CYP153-Familie. In einer bevorzugten Ausführungsform wird unter dem Begriff "Cytochrom P450-Monooxygenase der CYP153-Familie" eine cytosolische Oxidase verstanden, die Teil eines 3 Komponenten-Systems ist, das weiterhin ein Ferredoxin und eine Ferredoxin-Reduktase umfasst, mit einer Alkan-Bindestelle und der Fähigkeit, Alkane zu hydroxylieren. In einer besonders bevorzugten Ausführungsform handelt es sich um ein Enzym, das zu wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist oder um ein Enzym, das eine Polypeptidsequenz umfasst, die wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist und darüber hinaus Alkanhydroxylase-Aktivität aufweist. In einer bevorzugten Ausführungsform ist unter dem Begriff "Alkanhydroxylase-Aktivität", wie hierin verwendet, die Fähigkeit zu verstehen, die Hydroxylierung von Alkanen oder unsubstituierten linearen Alkylresten umfassend wenigstens fünf, bevorzugt zwölf Kohlenstoffstoffreste zu katalysieren. In einer weiteren bevorzugten Ausführungsform wird unter dem Begriff "Cytochrom P450-Monooxygenase der CYP153-Familie" eine nicht membrangebundene Oxidase verstanden, die eine Bindestelle für Alkane, unsubstituierte lineare Alkylreste umfassend wenigstens fünf, bevorzugt zwölf Kohlenstoffstoffreste oder einfach hydroxylierte Alkane und deren Polypeptidkette das Motiv LL(I/L)(V/I)GGNDTTRN umfasst. In einer bevorzugten Ausführungsform handelt es sich bei einer "Cytochrom P450-Monooxygenase der CYP153-Familie", wie hierin verwendet, um eine Cytochrom P450-Monooxygenase der CYP153-Familie aus *Alcanivorax borkumensis* SK2 (Datenbankcode YP_691921) oder eine Variante, die bevorzugt Alkanhydroxylaseaktivität aufweist.

Die Verwendung von Cytochrom P450-Monooxygenasen der CYP153-Familie zur Hydroxylierung von Alkanen ist im Stand der Technik beschrieben, ebenso Enzymtests zur Bestimmung der Enzymaktivität und Verfahren zur Expression und Aufreinigung (Scheps, D., Malca, H., Hoffmann, B., Nestl, B. M, und Hauer, B. (2011) Org. Biomol. Chem., 9, 6727). Neben einem zu oxidierenden Alkan oder unsubstituiertem linearen Alkylrest umfassend wenigstens fünf, bevorzugt zwölf Kohlenstoffstoffreste umfassen die für die Reaktion des Enzyms umfassenden Substrate Sauerstoff und Elektronen, die in Form von NADH bevorzugt über die anderen beiden Komponenten, Ferredoxin und eine Ferredoxin-Reduktase, auf die Oxidase übertragen werden. Scheps *et al.* (2011) sowie Roome, P. W., Jr., Philley, J. C., und Peterson (1983) J. Biol. Chem. 258, 2593, Roome, P.W., and Peterson, J. A. (1988), Arch. Biochem. Biophys., 266, 41 und Peterson, J. A., Lorence, M. C., und Amarneh, B. (1990) J. Biol. Chem, 265, 6066 offenbaren auch Verfahren zur Gewinnung von Ferredoxin und Ferredoxin-Reduktase in funktioneller Form.

Als Zelle wird in der Regel eine Zelle gewählt werden, die unter Verbrauch von Alanin ein Produkt von besonderem Interesse herzustellen vermag. Besonders vorteilhaft ist dafür die Verwendung einer rekombinanten Zelle. In einer bevorzugten Ausführungsform wird unter dem Begriff "rekombinant", wie hierin verwendet, verstanden, dass das als rekombinant bezeichnete, in die "rekombinante Zelle" eingebrachte, Nukleinsäuremolekül in dieser Form in der Natur nicht vorkommt" sondern ein unter Verwendung von molekularbiologischen oder chemischsynthetischen Verfahren hergestelltes Nukleinsäuremolekül ist bzw. dass die als rekombinant bezeichnete Zelle ein rekombinantes Nukleinsäuremolekül oder ein davon kodiertes Polypeptid umfasst, insbesondere ein Polypeptid mit Alanin-Dehydogenase-Aktivität. Molekularbiologische Routineverfahren zum Herstellen rekombinanter Nukleinsäuremoleküle und Zellen sind im Stand der Technik beschrieben, beispielsweise in Sambrook *et al.* (1989) oder Schlegl & Fuchs (2007).

Die Zelle stellt erfindungsgemäß unter Verbrauch ein Produkt von besonderem Interesse her. In einer bevorzugten Ausführungsform ist für diese Herstellung Alanin als Edukt an irgendeiner Stufe der Herstellung unerlässlich, beispielsweise für die Herstellung eines alaninhaltigen Polypeptides, im Gegensatz zur Herstellung eines Produktes, für dessen Herstellung zwar Kohlenstoff-, Wasserstoff- und Sauerstoffatome wie die aus dem Alanin erforderlich sind, die jedoch gleichermaßen aus anderen Quellen stammen können. Bevorzugt übersteigt die für die Synthese verbrauchte oder verbrauchbare Menge an Alanin die Menge, die die verwendete Zelle natürlich produzieren kann und besonders bevorzugt den oder zumindest einen die Ausbeute an dem interessierenden Produkt begrenzenden Faktor dar.

Besonders vorteilhaft ist es weiterhin, wenn die Zelle die Alanin-Dehydrogenase und/oder wenigstens ein weiteres Enzym überexprimiert. Dies kann erreicht werden, indem ein Vektor, der ein für das Enzym kodierende Nukleinsäuremolekül umfasst, durch Transformation o.ä. in die Zelle eingeschleust wird oder das für das Enzym kodierendes Nukleinsäuremolekül in die genetische Ausstattung der Zelle, beispielsweise ein Chromosom, eingebaut wird. Für zahlreiche biotechnologisch bedeutsame Arten von Zellen, z.B. *E. coli,* sind geeignete Verfahren und Vektoren bekannt, die für die Expression oder Überexpression eines Nukleinsäuremoleküls verwendet werden können, beispielsweise die Vektoren vom Typ pET oder pGEX und für deren Expression geeignete Zellen (Moffatt & Studier (1986), Rosenberg *et al.* (1987) und Studier *et al* (1990).

Die Lehre der vorliegenden Erfindung kann nicht nur unter Verwendung der bzw. auf die exakten Aminosäure- oder Nukleinsäuresequenzen der hierin beschriebenen biologischen Makromoleküle, beispielsweise einer Alanin-Dehydrogenase, ausgeführt bzw. angewendet werden, beispielsweise durch Knock out eines Gens, das für ein eine der Reaktionen der β-Oxidation katalysierendes Enzym kodiert, sondern auch unter Verwendung von bzw. auf Varianten derartiger Makromoleküle, die durch Deletion, Addition oder Substitution einer oder mehr als einer Aminosäuren oder Nukleinsäuren erhalten werden können. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder solche lediglich konservativ substituiert sind, beispielsweise ein Glutamat statt einem Aspartat oder ein Leucin statt einem Valin. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3rd edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatische Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den K_{M}- und/oder k_{cat}- Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Alanin-Dehydrogenase. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in Ausubel *et al. 1995* beschrieben. Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet in einer bevorzugten Ausführungsform unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996). Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Stringente Wasch-Bedingungen können beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) im Waschpuffer erreicht werden, wobei eine Temperatur von in der Reihenfolge zunehmender Bevorzugung ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2 x SSC oder 0,1 x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise in der Reihenfolge zunehmender Bevorzugung mindestens 70% oder mindestens 80% oder mindestens 90%, mindestens 91 %, mindestens 92%, mindestens 93%, mindestens 94%, mindestens 95%, mindestens 96%, mindestens 97%, mindestens 98% oder mindestens 99% Identität zur Sequenz des eingesetzten Nukleinsäuremoleküls. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

Bei Schritt c) des erfindungsgemäßen Verfahrens kommt es zum Kontaktieren des wässrigen Kulturmediums mit einer hydrophoben organischen Lösung. In einer bevorzugten Ausführungsform der vorliegenden Erfindung bedeutet der Begriff "Kontaktieren", wie hierin verwendet, dass wässriges Kulturmedium und organische Lösung direkt in Kontakt gebracht werden, ohne dass eine mechanische, für ein wässriges Kulturmedium und/oder eine hydrophobe organische Lösung unüberwindbare Barriere, beispielsweise eine anorganische Membran, zwischengeschaltet ist. Beispielsweise kann das wässrige Kulturmedium in einem Fermenter vorgelegt werden, und die organische Lösung wird in den gleichen Fermenter zu dem Kulturmedium gegeben, so dass sich beide Flüssigkeiten vermischen können. In einer bevorzugten Ausführungsform findet das Kontaktieren zumindest teilweise unter Rühren, dem Einströmen von Gas oder ähnlichen Maßnamen statt, die dazu geeignet sind, die Kontaktfläche beider Phasen zu vergrößern.

Beim Kontaktieren ist zu beachten, dass Schritt c) ausreichend lange und bei ausreichend bemessener Grenzfläche der zwei Phasen erfolgt, damit die Zellen ausreichend in Kontakt mit der hydrophoben Phase c) treten können. Das Kontaktieren dauert in einer bevorzugten Ausführungsform wenigstens 10, 20, 30, 60 Minuten oder 2, 4, 6, 12, 18 oder 24 Stunden an. Schritt c) kann sofort zu Beginn von Schritt b) vonstatten gehen, d. h. das Kontaktieren erfolgt während der Kultivierung, oder Schritt b) vorgeschaltet sein, d. h. die organische Lösung wird vor Beginn von Schritt b) entfernt, beispielsweise durch Abdekantieren oder Abzentrifugation und Extraktion der wässrigen Phase.

Für die erfindungsgemäße Lehre ist essentiell, dass Schritt b) unter aeroben Bedingungen erfolgt. In einer bevorzugten Ausführungsform wird unter "aeroben Bedingungen", wie hierein verwendet, verstanden, dass die wässrige Phase in Kontakt mit molekularem Sauerstoff, beispielsweise in Form von atmosphärischer Luft steht, und bevorzugt keine Maßnahmen ergriffen sind, um die Sauerstoffkonzentration in der wässrigen Phase zu verringern, beispielsweise das Verschließen des Reaktionsgefäßes gegen den Gasaustausch mit der sauerstoffhaltigen Umgebungsluft. In einer besonders bevorzugten Ausführungsform wird Sauerstoff, z. B. in Form von Luft, aktiv in die wässrige Phase eingeleitet, beispielsweise durch Belüftung und Rühren.

Bei einigen Substraten kann der Eintritt des Moleküls ins Innere des Ganzzellkatalysators für die Herstellung der erwünschten Substanz limitierend sein. Im Falle langkettiger Alkane und Derivate davon ist bevorzugt, dass der Ganzzellkatalysator ein AlkL-Polypeptid aufweist. In einer bevorzugten Ausführungsform handelt es sich bei einem "AlkL-Polypeptid", wie hierin verwendet, um ein Polypeptid, das über eine Länge von 230 aufeinander abfolgenden Aminosäuren eine wenigstens 80, bevorzugt 90, noch bevorzugter 90%ige Sequenzidentität zu AlkL aus *Pseudomonas putida* (Datenbankcode CAB69081) und bevorzugt die Fähigkeit aufweist, den Import von langkettigen Alkanen ins Innere einer Zelle zu unterstützen. In einer weiteren Ausführungsform handelt es sich bei einem "Polypeptid der AlkL-Familie", wie hierin verwendet, um ein in der äußeren Membran eines Gram-negativen Bakteriums lokalisiertes Polypeptid, welches das Sequenzmotiv DXWAPAXQ(V/A)GXR, aufweist, wobei X eine proteinogene Aminosäure darstellt, und bevorzugt zusätzlich zum AlkL aus *Pseudomonas putida* (Datenbankcode CAB69081) oder eine Variante davon ist. Beispielhafte Mitglieder der AlkL-Familie umfassen AlkL aus *Pseudomonas putida* (Datenbankcode CAB69081), *Marinobacter aquaeolei* VT8 (Datenbankcode YP_957722), *Oceanicaulis alexandrii* HTCC2633 (Datenbankcode ZP_00953584), *Marinobacter manganoxydans* Mnl7-9 (Datenbankcode ZP_09158756), *Caulobacter* sp. K31 (Datenbankcode YP_001672217), *Pseudomonas oleovorans* (Datenbankcode Q00595) und Varianten davon.

Das erfindungsgemäße Verfahren kann unter Verwendung üblicher hydrophober organischer Lösungsmittel durchgeführt werden, die bei Raumtemperatur flüssige substituierte und nichtsubstituierte Alkane, Cycloalkane, Cycloalkene, Aryle, Fettsäuren, Fettsäureester, Alkohole, Heterocycloalkane, Heterocycloalkene und Heteroaryle umfassen. Dem Fachmann sind zahlreiche Lösungsmittel bekannt, die verwendet werden können, um eine hydrophobe organische Lösung herzustellen. In einer bevorzugten Ausführungsform wird unter dem Begriff "hydrophob", wie hierin verwendet, die Eigenschaft einer Flüssigkeit verstanden, im flüssigen Zustand in Anwesenheit von einer flüssigen wässrigen Phase eine eigene, von der wässrigen Phase klar abgegrenzte flüssige Phase auszubilden. Bei letzterer kann es sich um eine zusammenhängende flüssige Phase oder um eine Emulsion handeln. In einer weiteren bevorzugten Ausführungsform wird mit dem Begriff "hydrophob", wie hierin verwendet, die Eigenschaft einer Verbindung verstanden, sich im Wesentlichen nicht in Wasser zu lösen. Schließlich wird der Begriff in einer weiteren bevorzugten Ausführungsform, wie hierin verwendet, derart verstanden, dass eine derartige bezeichnete Verbindung einen P-Wert (J. Sangster, Octanol-Water Partition Coefficients: Fundamentals and Physical Chemistry, Vol. 2 of Wiley Series in Solution Chemistry, John Wiley & Sons, Chichester, 1997) aufweist, dessen dekadischer Logarithmus größer 0, bevorzugter größer 0,5, noch bevorzugter größer 1 und am bevorzugtesten größer 2 ist. Bevorzugte organische Lösungsmittel umfassen, sind aber nicht beschränkt auf Lösungsmittel aus der Gruppe umfassend bei Raumtemperatur flüssige substituierte und nichtsubstituierte Alkane, Cycloalkane, Cycloalkene, Aryle, Fettsäuren, Fettsäureester, Alkohole, Heterocycloalkane, Heterocycloalkene und Heteroaryle umfasst. Die hydrophobe organische Lösung kann auch eine Mischung umfassend mehr als ein hydrophobes organisches Lösungsmittel sein. Auch hydrophobe organische Lösungsmittel, die *per se* nicht flüssig sind, eignen sich, sofern sie Teil eines Gemisches von Lösungsmitteln sind, das in seiner Gesamtheit flüssig ist.

Zu berücksichtigen ist dabei unter Umständen, dass zahlreiche Lösungsmittel auf stoffwechselaktive Zellen mehr oder weniger toxisch wirken. Soll die Zelle ihre Stoffwechselaktivität also wenigstens zeitweilig behalten, so sind entsprechende mäßig oder gar nicht toxisch wirkende Konzentrationen des hydrophoben organischen Lösungsmittels zu verwenden. In einer besonders bevorzugten Ausführungsform handelt es sich bei dem Lösungsmittel um eine gesättigte oder ungesättigte Fettsäure mit wenigstens acht, bevorzugt wenigstens zwölf Kohlenstoffatomen, beispielsweise um Laurinsäure, Ölsäure oder Erukasäure oder die Methylester davon. In einer weiteren bevorzugten Ausführungsform handelt es sich bei dem Lösungsmittel mit eine Fettsäure der Formel CH₃ - (CH₂)ₓ - COOH, wobei x 8, 9, 10, 11, 12, 13, 14, 15, 16, 17, 18, 19, 20, 21, 22 oder mehr sein kann. In einer weiteren bevorzugten Ausführungsform handelt es sich um eine ungesättigte Fettsäure mit einer Doppelbindung, besonders bevorzugt um eine an der Position 9, besonders bevorzugt Ölsäure. In einer weiteren besonders bevorzugten Ausführungsform handelt es sich bei dem Lösungsmittel um Hexansäure.

Soll die Zelle unter aeroben Bedingungen über einen längeren Zeitraum in Anwesenheit der organischen Phase kultiviert werden, so empfiehlt sich die Verwendung einer organischen Phase aus biokompatiblen hydrophoben Lösungsmitteln. Als solche eignet sich bevorzugt die Mischung des Methylesters einer gesättigten Fettsäure mit einer ungesättigten Fettsäure, besonders eine Mischung aus Laurinsäuremethylester und Ölsäure. Das Volumen- oder, im Falle der Verwendung wenigstens einer in reiner Form festen Fettsäure, Gewichtsverhältnis beträgt dabei bevorzugt 20 zu 80 bis 80 zu 20, besonders bevorzugt 30 zu 70 bis 70 zu 30, am bevorzugtesten 40 zu 60 bis 60 zu 40.

Das Volumen der hydrophoben organischen Lösung sollte so bemessen sein, dass die organische Phase leicht abgetrennt werden kann und beträgt in einer bevorzugten Ausführungsform 2 bis 98, bevorzugter 5 bis 95, noch bevorzugter 10 bis 40, am bevorzugtesten 20 bis 30 Prozent des Gesamtvolumens von wässrigem Kulturmedium und hydrophober organischer Lösung. Der Fachmann ist mit zahlreichen Verfahren zum Abtrennen einer organischen Phase von einer wässrigen Phase vertraut, beispielsweise Abdekantieren, Entfernen mittels Scheidetrichter, Abzentrifugieren und dergleichen.

Das Verfahren kann verwendet werden, um Fettsäuren oder deren Ester zunächst zu oxidieren und anschließend zu aminieren. Dazu eignet sich beispielsweise ein Enzymsystem, wie es in der internationalen Patentanmeldung WO 2009/077461 beschrieben ist. Bei der stoffwechselaktiven Zelle handelt es sich in diesem Fall um eine Zelle, die eine rekombinante Alkanhydroxylase und eine Transaminase aufweist, bevorzugt darüber hinaus wenigstens ein Enzym aus der Gruppe umfassend Alkoholdehydrogenase, Alanindehydrogenase und Lactamhydrolase. In einer bevorzugten Ausführungsform wird unter dem Begriff "Transaminase", wie hierin verwendet, ein Enzym verstanden, das die Übertragung von α-Aminogruppen von einem Donor-, bevorzugt einer Aminosäure, auf ein Akzeptormolekül katalysiert. Beispielsweise kann die Transaminase von *Chromobacterium violaceum* ATCC 12472 (Datenbankcode NP_901695) und Varianten davon verwendet werden. In einer bevorzugten Ausführungsform wird unter dem Begriff "Lactamhydrolase", wie hierein verwendet, ein Enzyme verstanden, das die Kondensation einer Carboxylgruppe mit einer Amingruppe des gleichen Moleküls intramolekular katalysiert. Ein beispielhaftes System und geeignete Enzyme sind in der EP11004029 beschrieben.

Zusätzlich zu einer oder statt einer Monooxygenase kann die für das erfindungsgemäße Verfahren geeignete Zelle auch eine Alkoholdehydrogenase aufweisen. In einer bevorzugten Ausführungsform wird unter dem Begriff "Alkoholdehydrogenase", wie hierin verwendet, wird ein Enzym verstanden, das einen Aldehyd bzw. Keton zu dem entsprechenden primären bzw. sekundären Alkohol oxidiert. Beispiele umfassend die Alkoholdehydrogenasen von *Ralstonia eutropha* (ACB78191.1), *Lactobacillus brevis* (YP_795183.1), *Lactobacillus kefiri* (ACF95832.1), aus Pferdeleber, von *Paracoccus pantotrophus* (ACB78182.1) und *Sphingobium yanoikuyae* (EU427523.1) sowie die jeweiligen Varianten davon.

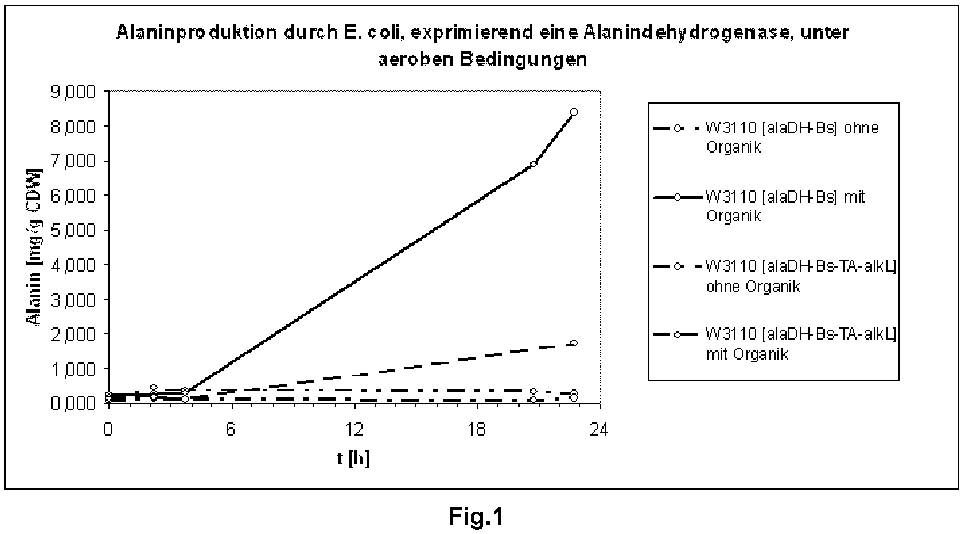

**Fig. 1** zeigt vergleichend die Alaninproduktion durch vier *E. coli*-Stämme, die sich dadurch unterscheiden, dass zwei Stämme AlkL exprimieren, in Anwesenheit und Abwesenheit von organischer Phase, wie es in Beispiel 1 beschrieben ist.

Die vorliegende Erfindung wird weiterhin durch die folgenden Figuren und nicht beschränkenden Beispiele veranschaulicht, denen weitere Merkmale, Ausführungsformen, Aspekte und Vorteile der vorliegenden Erfindung entnommen werden können.

### Beispiel: Alaninherstellung durch E. coli-Ganzzellkatalysator mit oder ohne AlkL-Expression verglichen in Anwesenheit und Abwesenheit einer organischen Phase

Die erhöhte Produktion von Alanin unter den besonderen Bedingungen dieser Erfindung wurde in einem 8-fach Parallelfermentationssystem der Firma DASGIP mit den Stämmen W3110 [alaDH_Bs] und W3110 [alaDH_Bs-TA-alkL] untersucht. Bei W3110 [alaDH_Bs] handelt es sich um einen Stamm von E. coli W3110, der ein pJ294-basiertes Plasmid ursprünglich von der Firma DNA2.0, mit dem Gen der Alanindehydrogenase aus *Bacillus subtilis,* umfasst. W3110 [alaDH_Bs-TA-alkL] ist ein Stamm, der ein pJ281-basiertes Plasmid, ursprünglich ebenfalls von der Firma DNA2.0, mit den Genen der oben genannten Alanindehydrogenase und einer Transaminase enthält, sowie ein weiteres pJ294-basiertes Plasmid mit dem Gen des Porins alkL (PCT/EP2011/053834, DE102011110945) beschrieben.

Für die Fermentation wurden 1L-Reaktoren verwendet. Die pH-Sonden wurden mittels einer Zwei-Punkt-Kalibration mit Maßlösungen von pH 4,0 und pH 7,0 kalibriert. Die Reaktoren wurden mit 300 mL Trinkwasser befüllt und 20 min bei 121 °C autoklaviert, um Sterilität zu gewährleisten. Anschließend wurden die pO2-Sonden über Nacht (mindestens 6 h lang) am DASGIP-System polarisiert. Am nächsten Morgen wurde das Wasser unter der Clean Bench entnommen und durch 300 mL Hochzelldichtemedium mit 100 mg/L Ampicillin ersetzt. Im Folgenden wurden die pO2-Sonden mit einer Einpunkt-Kalibration (Rührer: 400 rpm / Begasung: 10 sL/h Luft) kalibriert und die Feed-, Korrekturmittel- und Induktionsmittelstrecken mittels Clean-in-Place gereinigt. Dazu wurden die Schläuche mit 70% Ethanol, anschließend mit 1 M NaOH, dann mit sterilem VE-Wasser gespült und zuletzt mit den jeweiligen Medien befüllt.

Alanin produzierenden *E. coli*-Stämme wurden zuerst aus den jeweiligen Cryokulturen in LB-Medium (25 mL in einem 100 mL Schikanekolben) mit 100 mg/L Ampicillin über Nacht bei 37°C und 200 rpm ca. 18 h lang angezogen. Anschließend wurden je 2 mL der Kulturen in Hochzelldichtemedium (Glucose 15 g/L (30 mL / L einer separat autoklavierten 500 g/L Stammlösung mit 1% MgSO₄*7H₂O und 2,2% NH₄Cl), (NH₄)₂SO₄ 1,76 g/L, K₂HPO₄ 19,08 g/L, KH₂PO₄ 12,5 g/L, Hefeextrakt 6,66 g/L, Trinatriumcitrat-Dihydrat 2,24 g/L, Ammoniumeisencitrat-Lösung 17 mL/L einer separat autoklavierten 1% igen Stammlösung, Spurenelementlösung 5 mL/L separat autoklavierten Stammlösung (HCl (37 %) 36,50 g/L, MnCl₂*4H₂O 1,91 g/L, ZnSO₄*7H₂O 1,87 g/L, Ethylendiamintetraessigsäure-Dihydrat 0,84 g/L, H₃BO₃ 0,30 g/L. Na₂MoO₄*2H₂O 0,25 g/L, CaCl₂*2H₂O 4,70 g/L, FeSO₄*7H₂O 17,80 g/L, CuCl₂*2H₂O 0,15 g/L)) (Je Stamm 25mL in einem 100 mL Schikanekolben) mit 100 mg/L Ampicillin überimpft und bei 37°C /200 rpm weitere 5,5 h lang inkubiert.

Die optische Dichte der Kulturen bei 600 nm wurde bestimmt. Um die Reaktoren mit einer optischen Dichte von 0,1 anzuimpfen wurden entsprechende Mengen der Vorkultur in einer 5 mL Spritze unter sterilen Bedingungen aufgezogen und die Reaktoren mittels Kanüle durch ein mit 70% Ethanol überschichtetes Septum beimpft.

Folgendes Standardprogramm wurde verwendet:

| DO-Regler | | pH-Regler | |
|---|---|---|---|
| Preset | 0% | Preset | 0 ml/h |
| P | 0,1 | P | 5 |
| Ti | 300 s | Ti | 200 s |
| Min | 0% | Min | 0 mlL/h |
| Max | 100% | Max | 40 mL/h |

| N (Rotation) | von | bis | XO2 (Gasmischung) | von | bis | F (Gasfluss) | von | bis |
|---|---|---|---|---|---|---|---|---|
| Während des gesamten Prozesses | 0% | 30% | Während des gesamten Prozesses | 0% | 100% | Während des gesamten Prozesses | 15% | 80% |
| | 400 rpm | 1500 rpm | | 21 % | 21% | | 6 sL/h | 72 sL/h |

| Script | |
|---|---|
| Trigger scharf | 31% DO (1/60h) |
| Induktion IPTG | 2 h nach Feedstart |
| Feedtrigger | 50% DO |
| Feedrate | 3 [mL/h] |

Das durchgeführte Experiment lässt sich in zwei Phasen gliedern, die Anzucht, bei der die Zellen eine bestimmte optische Dichte erreichen sollen, und die nachfolgende Alaninproduktion, in der nach Zugabe der organischen Phase bestehend aus 25 % (w/w) Laurinsäuremethylester und 75 % (w/w) Ölsäure die Produktion des Alanins von in der Expression gebildeten Enzyme stattfinden soll. Entsprechende Versuche ohne organische Phase dienten hierbei als Kontrollen. Die pH-Werte wurden einseitig mit Ammoniak (12,5 %) auf pH 6,8 geregelt. Während Anzucht und Biotransformation wurde der gelöste Sauerstoff (DO, dissolved oxygen) in der Kultur über Rührerdrehzahl und Begasungsrate bei 30% geregelt.
Die Fermentation wurde als Feed-Batch durchgeführt, wobei der Feedstart, 5 g/Lh Glucosefeed (500 g/L Glucose mit 1% MgSO₄*7H₂O und 2,2% NH₄Cl, über einen DO-Peak getriggert wurde. Mit Feedstart wurde auch die Temperatur von vorher 37°C auf 30°C gesenkt. Die Expression der Alanindehydrogenase (und der anderen rekombinant eingebrachten Proteine) wurde 2 h nach Feedstart durch die automatische Zugabe von IPTG (1 mM Endkonzentration) induziert. Vor Start der von der organischen Phase induzierten Alaninproduktion wurde die optische Dichte der Kulturbrühen bestimmt.

Der Start der Alaninproduktion erfolgte 14 h nach Feedstart. Dazu wurden 150 mL eines Gemisches aus Laurinsäuremethylesther und Ölsäure (techn. 90%) als Batch zu der Fermentationsbrühe gegeben. Um ausreichend Ammonium-Ionen für die Alaninproduktion zur Verfügung zu haben, wurde eine halbe Stunde vor Zugabe der Organik 5 mL einer 3 M Ammoniumsulfatlösung zur Fermentationsbrühe gegeben. Zur Probennahme wurden 2 mL Fermentationsbrühe aus dem Kessel entnommen und sofort mit der Quenching-Lösung (40% (v/v) Ethanol; 0,8% (w/v) NaCl; -20°C) vermischt. Anschließend wurden die Proben bei 0°C / 5100 rpm für 10 min zentrifugiert. Der Überstand wurde verworfen und das Zellpellet 2 mL Methanol (-20°C) resuspendiert. Mit Hilfe dieser Proben wurde die Alaninkonzentration innerhalb der Zellen bestimmt.

Die Bestimmung von Alanin erfolgt mittels HPLC/UV Messung, nach Derivatisierung mittels ortho-Phthaldialdehyd. Es wurde der methanolische Überstand vermessen. Die wichtigsten chromatographischen Parameter sind in der nachfolgenden Tabelle zusammen gefasst.

| | |
|---|---|
| Säule | Luna 5u C8, 100 A, 150 X 4.60 mm , Firma Phenomenex |
| HPLC Anlage | Agilent 1200 |
| Laufmittel A | 2.5 mL Essigsäure (100 %) auf 1 L bidest. Wasser, pH Einstellung mit Natronlauge auf pH 6.0 |
| Laufmittel B | Methanol |
| Säulentemp. | 40 °C |
| Fluss | 1 mL/min |
| Gradient | 0.0-1 min: 30.0% B, 1.0-17.0 min: 90.0% B, 17-19.5 min: 90.0% B, 19.6-20.5 min: 30.0% B |
| Detektor | DAD, 334 nm |
| Derivatisierung/ Injektionsvolumen | Automatische Derivatisierung mittels Injektorprogramm, 1 µL Probe wird mit 9 µL Derivatisierungsreagenz umgesetzt; Zusammensetzung Derivatisierungsreagenz: 10 g/L o-Phthaldialdehyd gelöst in Boratpuffer (0.4 mol/L), unter Zugabe von Mercaptoethanol (5 mL/L) und Methanol (100 mL/L) |
| Kalibrierung | Externe Kalibrierung, Messbereich 50-2000 mg/L, 5-Punkte Kalibrierung, Kalibrierung vor und nach der Probenserie, Mittelung über beiden Kalibrierreihen, quadratische Regression |

Proben wurden 15 Minuten vor Organik-Zugabe sowie 2 h, 3.5 h, 20.5 h und 22.5 h nach Organikzugabe von allen Reaktoren genommen. Die Umsatzraten für Sauerstoff (OTR = oxygen transfer rate) und Kohlenstoff (CTR = carbon dioxide transfer rate) wurden während der Fermentation über die Abgasanalytik an den DASGIP-Systemen bestimmt. Die Fermentation wurde 23 h nach Start der Biotransformation beendet. Der Rührer, die Begasung, die Temperaturregelung und pH-Regelung wurden abgestellt und die Kessel 5-10 Minuten ruhig stehen gelassen.

### Ergebnisse:

Nach Zugabe der Organischen Phase zeigt sich in beiden untersuchten Fällen eine deutliche Zunahme der Alaninkonzentration in den verwendeten Zellen mit oder ohne AlkL-Expression. In den Vergleichsexperimente ohne Organikzugabe werden jeweils deutlich geringere Alaninkonzentrationen gemessen, die auch keinen aufsteigenden Trend aufweisen. (siehe **Fig. 1**)

## Patentansprüche

1. Verfahren zur Herstellung von Alanin umfassend
a) Bereitstellen einer eine rekombinante Alanin-Dehydrogenase exprimierenden Zelle,
b) Kultivieren der Zelle unter aeroben Bedingungen in Anwesenheit einer anorganischen Stickstoffquelle in einer wässrigen Phase, und
c) Kontaktieren der Zelle mit einer hydrophoben organischen Phase,
wobei es sich bei der Zelle um eine prokaryontische oder eine niedere eukaryontische Zelle handelt.

2. Verfahren nach Anspruch 1, wobei die Zelle eine Nukleinsäure umfassend eine für ein alkL-Polypeptid, bevorzugt das AlkL von *Pseudomonas putida* (Datenbankcode AJ245436) oder eine Variante davon kodierende Sequenz und/oder das alkL-Polypeptid oder eine Variante davon aufweist.

3. Verfahren nach Anspruch 2, wobei das alkL-Polypeptid oder die Variante davon heterolog ist und bevorzugt überexprimiert wird.

4. Verfahren nach einem der Ansprüche 1 bis 3, wobei Schritt c) dadurch verwirklicht wird, dass Schritt b) in Anwesenheit der organischen Phase durchgeführt wird.

5. Verfahren nach einem der Ansprüche 1 bis 4, wobei die organische Phase einen hydrophoben Fettsäureester umfasst, bevorzugt Laurinsäuremethylester.

6. Verfahren nach Anspruch 5, wobei die organische Phase neben dem hydrophoben Fettsäureester eine hydrophobe Fettsäure umfasst.

7. Verfahren nach Anspruch 6, wobei die organische Phase wenigstens ein hydrophobes Lösungsmittel aus der Gruppe umfassend unsubstituierte, substituierte, verzweigte und unverzweigte Alkane, Cycloalkane, Aryle, Heteroaryle, Dialkylether, Fettalkohole, Triglyceride und Halogenkohlenwasserstoff umfasst.

8. Verfahren nach Anspruch 6, wobei die hydrophobe Fettsäure eine ungesättigte Fettsäure ist, bevorzugt Ölsäure.

9. Verfahren nach einem der Ansprüche 1 bis 3, wobei die Zelle eine heterologe Transaminase aufweist, die Alanin als Substrat erkennt, und die Transaminase bevorzugt aus der Gruppe ausgewählt ist, die die ω-Transaminase aus *Chromobacterium violaceum* ATCC 12472 (Datenbankcode NP_901695) sowie Varianten davon umfasst.

10. Verfahren nach Anspruch 9, wobei die Zelle zusätzlich zur heterologen Transaminase eine oder mehr als eine Monooxygenase aufweist, die allein oder sequentiell die Oxidation einer Fettsäure oder eines Fettsäureesters zur ω-Oxo-Fettsäure oder zum ω-Oxo-Fettsäureester katalysiert.

11. Verfahren nach Anspruch 10, wobei die eine oder mehr als eine Monooxygenase heterolog ist und bevorzugt aus der Gruppe ausgewählt ist, die Monooxygenasen aus der AlkB-Familie und Cytochrom P450-Monoxygenasen der CYP153-Familie und Varianten davon umfasst.

12. Verfahren nach einem der Ansprüche 1 bis 10, wobei es sich bei der Zelle um eine bakterielle Zelle handelt, bevorzugt um *Escherichia coli.*

13. Verfahren nach einem der Ansprüche 1 bis 12, wobei Schritt c) wenigstens 60 Minuten andauert.

14. Verfahren nach einem der Ansprüche 1 bis 13, wobei die wässrige Phase weniger als 10 mM, bevorzugt weniger als 5 mM, noch bevorzugter noch weniger als 1 mM Alanin umfasst.

15. Verfahren nach einem der Ansprüche 1 bis 14, wobei die organische Phase wenigstens 5 Volumenprozent, bevorzugt mehr als 20 Volumenprozent der Summe der Volumina von wässriger und organischer Phase ausmacht.

16. Verwendung einer eine heterologe Alanin-Dehydrogenase exprimierenden Zelle, zur Herstellung von Alanin unter aeroben Bedingungen in Anwesenheit einer hydrophoben organischen Phase, wobei es sich bei der Zelle um eine prokaryontische oder eine niedere eukaryontische Zelle handelt und wobei die organische Phase bevorzugt einen hydrophoben Fettsäureester und eine hydrophobe Fettsäure, noch bevorzugter Laurinsäuremethylester und Ölsäure umfasst.

17. Verwendung einer hydrophoben organischen Phase zur Erhöhung der Ausbeute an Alanin umfassend Kontaktieren einer die Alanin herstellenden prokaryontischen oder einer niederen eukaryontischen Zelle mit der organischen Phase, wobei die organische Phase bevorzugt einen hydrophoben Fettsäureester und eine hydrophobe Fettsäure, noch bevorzugter Laurinsäuremethylester und Ölsäure umfasst.

18. Verfahren nach einem der Ansprüche 1 bis 15, wobei die Zelle in einer wässrigen Lösung bei einer optischen Dichte von wenigstens 0,5, bevorzugter wenigstens 1, noch bevorzugter wenigstens 5, am bevorzugtesten wenigstens 10 vorliegt.

19. Verfahren nach einem der Ansprüche 1 bis 15, wobei es sich bei der Alanin-Dehydrogenase um die Alanin-Dehydrogenase aus *Bacillus subtilus* (Datenbankcode P_391071) oder um eine Variante davon handelt.

## Claims

1. Process for the production of alanine, which process comprises
a) providing a cell which expresses a recombinant alanine dehydrogenase,
b) culturing the cell under aerobic conditions in an aqueous phase in the presence of an inorganic nitrogen source, and
c) bringing the cell into contact with a hydrophobic organic phase,
wherein the cell is a prokaryotic or a lower eukaryotic cell.

2. Process according to Claim 1, wherein the cell includes a nucleic acid comprising a sequence encoding an alkL polypeptide, preferably the *Pseudomonas putida* AlkL (database code AJ245436) or a variant thereof, and/or the alkL polypeptide or a variant thereof.

3. Process according to Claim 2, wherein the alkL polypeptide or the variant thereof is heterologous and is preferably overexpressed.

4. Process according to one of Claims 1 to 3, wherein step c) is carried out by carrying out step b) in the presence of the organic phase.

5. Process according to one of Claims 1 to 4, wherein the organic phase comprises a hydrophobic fatty acid ester, preferably methyl laurate.

6. Process according to Claim 5, wherein the organic phase comprises a hydrophobic fatty acid beside the hydrophobic fatty acid ester.

7. Process according to Claim 6, wherein the organic phase comprises at least one hydrophobic solvent selected from the group comprising unsubstituted, substituted, branched and unbranched alkanes, cycloalkanes, aryls, heteroaryls, dialkyl ethers, fatty alcohols, triglycerides and halohydrocarbon.

8. Process according to Claim 6, wherein the hydrophobic fatty acid is an unsaturated fatty acid, preferably oleic acid.

9. Process according to one of Claims 1 to 3, wherein the cell includes a heterologous transaminase which recognizes alanine as a substrate, and which transaminase is preferably selected from the group which comprises the ω-transaminase from *Chromobacterium violaceum* ATCC 12472 (database code NP_901695) and variants thereof.

10. Process according to Claim 9, wherein the cell includes, in addition to the heterologous transaminase, one or more than one monooxygenase which, alone or in sequence, catalyses the oxidation of a fatty acid or of a fatty acid ester to give the ω-oxo-fatty acid or the ω-oxo-fatty acid ester.

11. Process according to Claim 10, wherein the one or more than one monooxygenase is heterologous and preferably selected from the group which comprises monooxygenases from the AlkB family and cytochrome P450 monooxygenases from the CYP153 family and variants thereof.

12. Process according to one of Claims 1 to 10, wherein the cell is a bacterial cell, preferably *Escherichia coli.*

13. Process according to one of Claims 1 to 12, wherein step c) takes at least 60 minutes.

14. Process according to one of Claims 1 to 13, wherein the aqueous phase comprises less than 10 mM, preferably less than 5 mM, more preferably even less than 1 mM alanine.

15. Process according to one of Claims 1 to 14, wherein the organic phase amounts to at least 5 percent by volume, preferably more than 20 percent by volume, of the total of the volumes of aqueous and organic phase.

16. Use of a cell which expresses a heterologous alanine dehydrogenase for the production of alanine under aerobic conditions in the presence of a hydrophobic organic phase, wherein the cell is a prokaryotic or a lower eukaryotic cell and wherein the organic phase preferably comprises a hydrophobic fatty acid ester and a hydrophobic fatty acid, even more preferably methyl laurate and oleic acid.

17. Use of a hydrophobic organic phase for increasing the yield of alanine, comprising the bringing into contact of a prokaryotic or lower eukaryotic cell which produces the alanine with the organic phase, wherein the organic phase preferably comprises a hydrophobic fatty acid ester and a hydrophobic fatty acid, even more preferably methyl laurate and oleic acid.

18. Process according to one of Claims 1 to 15, wherein the cell is present in an aqueous solution at an optical density of at least 0.5, more preferably at least 1, even more preferably at least 5, most preferably at least 10.

19. Process according to one of Claims 1 to 15, wherein the alanine dehydrogenase is the alanine dehydrogenase from *Bacillus subtilus* (database code NP_391071) or a variant thereof.

## Revendications

1. Procédé de fabrication d'alanine, comprenant :
a) la préparation d'une cellule exprimant une alanine déshydrogénase recombinante,
b) la culture de la cellule en conditions aérobies en présence d'une source d'azote inorganique dans une phase aqueuse, et
c) la mise en contact de la cellule avec une phase organique hydrophobe,
la cellule étant une cellule procaryote ou une cellule eucaryote inférieure.

2. Procédé selon la revendication 1, dans lequel la cellule comprend un acide nucléique comprenant une séquence codant pour un polypeptide alkL, de préférence l'alkL de *Pseudomonas putida* (code de banque de données AJ245436) ou une variante de celui-ci, et/ou le polypeptide alkL ou une variante de celui-ci.

3. Procédé selon la revendication 2, dans lequel le polypeptide alkL ou la variante de celui-ci est hétérologue et est de préférence surexprimé.

4. Procédé selon l'une quelconque des revendications 1 à 3, dans lequel l'étape c) a lieu en réalisant l'étape b) en présence de la phase organique.

5. Procédé selon l'une quelconque des revendications 1 à 4, dans lequel la phase organique comprend un ester d'acide gras hydrophobe, de préférence l'ester méthylique de l'acide laurique.

6. Procédé selon la revendication 5, dans lequel la phase organique comprend un acide gras hydrophobe en plus de l'ester d'acide gras hydrophobe.

7. Procédé selon la revendication 6, dans lequel la phase organique comprend au moins un solvant hydrophobe du groupe comprenant les alcanes, les cycloalcanes, les aryles, les hétéroaryles, les éthers dialkyliques, les alcools gras, les triglycérides et les hydrocarbures halogénés non substitués, substitués, ramifiés et non ramifiés.

8. Procédé selon la revendication 6, dans lequel l'acide gras hydrophobe est un acide gras insaturé, de préférence l'acide oléique.

9. Procédé selon l'une quelconque des revendications 1 à 3, dans lequel la cellule comprend une transaminase hétérologue qui reconnaît l'alanine en tant que substrat, et la transaminase est de préférence choisie dans le groupe comprenant la transaminase ω) de *Chromobacterium violaceum* ATCC 12472 (code de banque de données NP_901695) et les variantes de celle-ci.

10. Procédé selon la revendication 9, dans lequel la cellule comprend en plus de la transaminase hétérologue une ou plusieurs monooxygénases, qui catalysent seules ou séquentiellement l'oxydation d'un acide gras ou d'un ester d'acide gras en l'acide oxo-gras ω ou en l'ester d'acide oxo-gras ω).

11. Procédé selon la revendication 10, dans lequel la ou les monooxygénases sont hétérologues et sont de préférence choisies dans le groupe comprenant les monooxygénases de la famille AlkB et les monooxygénases du cytochrome P450 de la famille CYP153 et les variantes de celles-ci.

12. Procédé selon l'une quelconque des revendications 1 à 10, dans lequel la cellule est une cellule bactérienne, de préférence *Escherichia coli.*

13. Procédé selon l'une quelconque des revendications 1 à 12, dans lequel l'étape c) dure au moins 60 minutes.

14. Procédé selon l'une quelconque des revendications 1 à 13, dans lequel la phase aqueuse comprend moins de 10 mM, de préférence moins de 5 mM, de manière davantage préférée moins de 1 mM d'alanine.

15. Procédé selon l'une quelconque des revendications 1 à 14, dans lequel la phase organique représente au moins 5 pour cent en volume, de préférence plus de 20 pour cent en volume, de la somme des volumes de la phase aqueuse et de la phase organique.

16. Utilisation d'une cellule exprimant une alanine déshydrogénase hétérologue pour la fabrication d'alanine en conditions aérobies en présence d'une phase organique hydrophobe, la cellule étant une cellule procaryote ou une cellule eucaryote inférieure et la phase organique comprenant de préférence un ester d'acide gras hydrophobe et un acide gras hydrophobe, de manière davantage préférée l'ester méthylique de l'acide laurique et l'acide oléique.

17. Utilisation d'une phase organique hydrophobe pour augmenter le rendement en alanine, comprenant la mise en contact d'une cellule procaryote ou eucaryote inférieure produisant l'alanine avec la phase organique, la phase organique comprenant de préférence un ester d'acide gras hydrophobe et un acide gras hydrophobe, de manière davantage préférée l'ester méthylique de l'acide laurique et l'acide oléique.

18. Procédé selon l'une quelconque des revendications 1 à 15, dans lequel la cellule se présente dans une solution aqueuse à une densité optique d'au moins 0,5, de préférence d'au moins 1, de manière davantage préférée d'au moins 5, de manière préférée entre toutes d'au moins 10.

19. Procédé selon l'une quelconque des revendications 1 à 15, dans lequel l'alanine déshydrogénase est l'alanine déshydrogénase de *Bacillus subtilus* (code de banque de données NP_391071) ou une variante de celle-ci.
